# FASCICULE DE BREVET EUROPEEN

(11) **EP 0 500 475 B1**
(45) Date de publication et mention de la délivrance du brevet: **15.03.1995**
(21) Numéro de dépôt: 92420034.8
(22) Date de dépôt: 28.01.1992
(51) Int. Cl.: G01P 13/00, A61M 5/168

(54) **Détecteur d'écoulement d'un liquide dans un récipient**
Detektor für Flüssigkeitsströmung in einem Behälter
Sensor for flow of liquid in a receptacle

(30) Priorité: 22.02.1991 FR 9102380
(43) Date de publication de la demande: 26.08.1992
(73) Titulaire: HOSPAL INDUSTRIE, F-69883 Meyzieu Cédex (FR)
(72) Inventeur: Chevallet, Jacques, F-69360 Serezin du Rhone (FR); Riquier, Jean-Claude, F-69140 Rilleux (FR)

(56) Documents cités:
- EP-A- 0 128 708
- DE-U- 8 627 669
- FR-A- 2 012 750
- GB-A- 1 088 747

## Description

La présente invention concerne un détecteur d'écoulement d'un liquide dans un récipient. Ce détecteur trouve une application particulière dans le domaine médical comme détecteur d'écoulement d'un liquide médical dans un piège à bulles d'un circuit de perfusion ou d'un circuit de circulation extracorporelle de sang.

L'interruption involontaire de l'infusion d'un liquide médical à un patient en cours de traitement, quand elle n'est pas détectée rapidement, peut avoir des conséquences graves, notamment pour les grands brûlés, qui perdent beaucoup d'eau plasmatique et doivent être perfusés en permanence, et pour les patients soumis à un traitement d'épuration du sang par hémofiltration ou hémodiafiltration auxquels des quantités importantes de plasma sanguin sont soutirées par ultrafiltration et sont compensées par une infusion simultanée d'un liquide de substitution.

Une telle interruption peut avoir des causes multiples : oubli d'un clamp sur une canalisation du circuit de liquide médical, arrêt de la pompe de circulation utilisée, le cas échéant, pour en provoquer l'écoulement et en réguler le débit, poche de liquide médical arrivée en fin de vidage, etc.

Deux moyens de détection d'écoulement sont utilisés couramment, seuls ou en combinaison, pour détecter l'interruption involontaire de l'infusion d'un liquide médical à un patient. L'un de ces moyens est constitué par un détecteur de rotation de la pompe de circulation du liquide médical et le second de ces moyens est constitué par un capteur de pression dans un piège à bulles qui est généralement prévu sur les circuits de perfusion. Cependant, ni l'un ni l'autre de ces moyens de détection n'est universel et, même en combinaison, ils peuvent être pris en défaut : le détecteur de rotation de la pompe de circulation du liquide médical ne permet évidemment pas de mettre en évidence la présence d'un clamp sur le circuit de liquide médical, et le capteur de pression ne permet pas non plus de détecter la présence d'un clamp sur le circuit entre le piège à bulles et la pompe de circulation, la pression dans ce cas ne variant sensiblement pas dans le piège à bulles.

Le document FR-A-2 012 750 décrit un dispositif indicateur du débit de lait dans une tubulure collectrice alimentée par plusieurs trayeuses. Ce dispositif comprend une chambre allongée de section droite annulaire, dans laquelle s'écoule le lait provenant d'une trayeuse. La chambre, qui a une paroi extérieure transparente, est montée en saillie sur la tubulure avec laquelle elle communique par plusieurs orifices calibrés de diamètres croissants ménagés à différents niveaux sur une paroi intérieure de la chambre. Une sonde détectrice s'étend dans la chambre, du sommet de la chambre jusqu'à au-dessus de l'orifice inférieur.

Ce dispositif indique le débit du lait s'écoulant dans la tubulure par la hauteur du lait dans la chambre, visible au travers de la paroi extérieure transparente. Lorsque le débit est inférieur au débit autorisé par l'orifice inférieur, le lait ne monte pas dans la chambre jusqu'au niveau de la sonde détectrice, et une alarme est alors émise.

Un but de l'invention est de réaliser un détecteur d'écoulement de liquide dans un récipient qui soit parfaitement fiable et, par là, particulièrement adapté à un usage médical.

Pour atteindre ce but, on prévoit, conformément à l'invention, un dispositif de détection de l'écoulement d'un liquide dans un récipient à usage médical, comprenant des moyens de détection de la présence de liquide dans des moyens de retenue de liquide ayant un orifice d'évacuation à leur base, caractérisé en ce que les moyens de retenue sont situés à l'intérieur du récipient entre un orifice d'entrée de liquide dans le récipient et le fond du récipient et en ce que les moyens de détection sont disposés à l'extérieur du récipient.

Grace à cette disposition, la détection de la présence de liquide dans les moyens de retenue est toujours significative d'un écoulement de liquide dans le récipient, pourvu que le liquide ne soit pas susceptible de monter dans le récipient jusqu'au niveau du détecteur de liquide. Par ailleurs, par rapport à une détection qui se ferait au niveau ou dans le prolongement de l'orifice d'entrée du liquide dans le récipient, sur une veine liquide plus ou moins irrégulière selon le débit, une détection qui s'effectue sur un liquide stabilisé, dans ou en sortie des moyens de retenue, présente l'avantage d'être fiable tout en ne mettant en oeuvre que des moyens très simples, quel que soit le débit d'entrée du liquide dans le récipient.

Selon une caractéristique de l'invention, le dispositif comprend en outre des moyens de détection de la présence de liquide dans le récipient, à un niveau situé au-dessous du niveau des moyens de détection de la présence de liquide dans les moyens de retenue.

Ces second moyens de détection sont utiles, comme sécurité, dans le cas de récipients de faible volume, tels que les pièges à bulles des circuits de circulation extracorporelle de sang, où le liquide est susceptible de monter jusqu'au niveau des moyens de détection de la présence de liquide dans les moyens de retenue.

Selon une autre caractéristique de l'invention, le dispositif comprend des moyens d'alarme pour recevoir des signaux émis par le détecteur de la présence de liquide dans les moyens de retenue et des signaux émis par le détecteur de la présence de liquide dans le récipient, et pour commander une alarme quand aucun des détecteurs ne détecte la présence de liquide ou quand le détecteur de présence de liquide dans le récipient détecte la présence de liquide.

Dans un mode de réalisation particulier de l'invention, les moyens de détection de la présence de liquide dans les moyens de retenue sont disposés au-dessous du niveau de l'orifice d'évacuation des moyens de retenue, lequel est disposé contre une paroi latérale du récipient.

Avantageusement, les moyens de détection de la présence de liquide dans les moyens de retenue et/ou le réservoir sont des moyens de détection optique. Ces moyens de détection optique peuvent comprendre une source et un récepteur de lumière situés à l'extérieur du récipient en vis -à-vis d'une paroi transparente de ce récipient et disposés l'un par rapport à l'autre de façon qu'un rayon émis par la source de lumière se réfléchisse sur la surface interne de cette paroi en direction du récepteur lorsque cette surface n'est pas baignée par un liquide et, lorsque cette surface est baignée par un liquide, soit réfracté dans ce liquide.

D'autres caractéristiques et avantages de l'invention apparaîtront à la lecture de la description qui va suivre. On se reportera aux dessins annexés sur lesquels :
La figure 1 est une vue en coupe schématique, selon un plan vertical, d'un récipient équipé d'un premier mode de réalisation du détecteur d'écoulement selon l'invention ;
La figure 2 est une vue en coupe schématique, selon la ligne A-A, du récipient de la figure 1 ; et
La figure 3 est une vue en coupe schématique, selon un plan vertical, d'un récipient équipé d'un second mode de réalisation du détecteur d'écoulement selon l'invention.

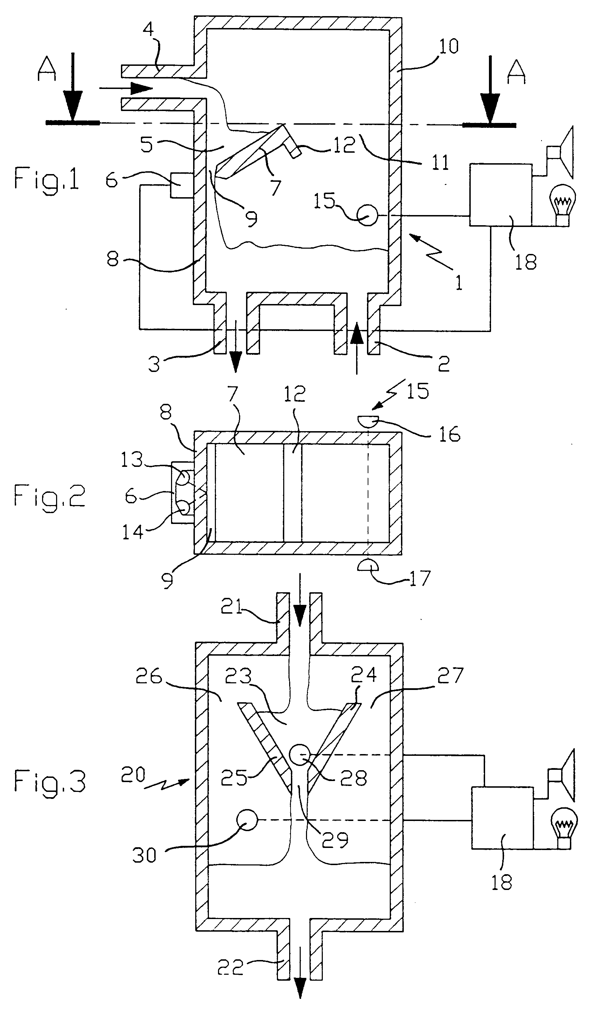

Le récipient représenté sur les figures 1 et 2 est un piège à bulles 1 de forme parallélépipédique rectangle faisant partie d'un circuit de circulation extracorporelle de sang. A sa base sont connectées deux canalisations 2, 3 par lesquelles le sang pénètre dans le piège à bulles 1 et en sort, respectivement. Par ailleurs, le piège à bulles 1 est relié par une canalisation 4 connectée à sa partie supérieure à une source de liquide médical, une poche par exemple (non représentée). Dans cet exemple, le piège à bulles 1 est donc situé à l'extrémité terminale du circuit de liquide médical.

Conformément à l'invention, le piège à bulles 1 est muni d'un détecteur d'écoulement du liquide médical comprenant des moyens de retenue 5 pour le liquide, associés à des moyens de détection 6 de la présence de liquide dans les moyens de retenue. Les moyens de retenue comprennent une paroi 7 intérieure transversale, inclinée par rapport à la paroi latérale 8 sur laquelle débouche la canalisation 4, et disposée au-dessous du niveau de la canalisation 4. La paroi inclinée 7 n'est pas jointive à la paroi latérale 8 de sorte que la retenue qu'elle délimite dans le piège à bulles 1 est ouverte à son extrémité inférieure. L'orifice d'évacuation 9 ainsi formé est calibré pour permettre la formation d'un film de liquide régulier sur la paroi latérale 8 lorsque le piège à bulles 1 est alimenté en liquide médical. La paroi inclinée 7 n'est pas non plus jointive à la paroi latérale 10 du piège à bulles opposée à la paroi 8 de façon qu'elle délimite avec celle-ci une ouverture de trop-plein 11. Le bord supérieur de la cloison inclinée 7 est muni d'un déflecteur 12 destiné à empêcher le liquide qui déborde des moyens de retenue 5 lorsque le débit d'arrivée du liquide est supérieur au débit de fuite de l'ouverture 9, de couler le long de la face inférieure de la paroi 7 et de venir perturber le film de liquide formé sur la paroi latérale 8 (ce déflecteur pourrait naturellement être disposé le long du bord inférieur de la paroi 7).

Les moyens de détection 6 de la présence de liquide dans les moyens de retenue 5 sont constitués par un détecteur optique prévu pour détecter l'existence d'un film de liquide sur la paroi latérale 8. Ce détecteur est donc disposé au-dessous du niveau de l'orifice d'évacuation 9 des moyens de retenue, sur la face extérieure de la paroi 8 qui, au moins dans cette zone, est transparente. Le détecteur optique comprend une source de lumière 13, telle qu'une diode électroluminescente et un détecteur de lumière 14, tel qu'un phototransistor, qui sont disposés symétriquement par rapport à la surface intérieure de la paroi 8 de façon que, lorsque cette surface est sèche, la lumière émise par la source 13 est intégralement réfléchie en direction du détecteur 14 et que, lorsque cette surface est baignée par un film de liquide, la lumière émise par la source 13 est réfractée en grande partie dans le liquide, le détecteur 14 ne recevant plus alors qu'une faible quantité de lumière réfléchie.

Un détecteur 15 de la présence de liquide dans le piège à bulles 1 est disposé sur les parois du piège à bulles 1 perpendiculaires à la paroi 8, au-dessous du niveau du détecteur 6. Le détecteur 15 de la présence de liquide dans le piège à bulles 1 est situé à un niveau que le sang n'atteint pas dans les conditions de fonctionnement normal du circuit de circulation extracorporelle de sang. Ce détecteur est également un détecteur optique qui est constitué par une source et un détecteur de lumière 16, 17 disposés en vis-à-vis, à l'extérieur des parois perpendiculaires à la paroi 8, lesquelles sont transparentes au moins sur une zone en regard de cette source et ce détecteur de lumière.

Les signaux émis par les deux détecteurs optiques 6 et 15 sont reçus et traités dans un circuit d'alarme 18 qui commande une alarme sonore ou lumineuse soit lorsque le détecteur 6 ne détecte pas de liquide soit lorsque le détecteur 15 détecte du liquide.

En fonctionnement normal du circuit de circulation extracorporelle de sang comprenant le piège à bulles 1, un flux continu de sang entretenu par une pompe de circulation (non représentée) traverse le piège à bulles 1, et le niveau du sang dans le piège à bulles 1, quine varie sensiblement pas, reste au-dessous du niveau du détecteur 15. Par ailleurs, un liquide médical provenant d'une poche et, le cas échéant, mis en circulation à l'aide d'une pompe (non représentées), s'écoule continûment par la canalisation 4 dans le piège à bulles 1 où, selon son débit d'entrée, il remplit plus ou moins la retenue 5, dont il s'échappe par l'orifice d'évacuation 9 pour former un film sur la paroi 8 et finir par se mélanger au sang présent dans le piège à bulles 1.

Dans ces conditions normales de fonctionnement, le détecteur 6 détecte la présence de liquide le long de la paroi 8 et le signal qu'il émet est interprété par le circuit d'alarme 18 comme significatif de l'écoulement du liquide médical dans le piège à bulles 1 parce que, simultanément, ce circuit prend en compte le signal émis par le détecteur 15, qui indique alors l'absence de liquide à son niveau. De fait, le signal du détecteur 15 correspondant à une absence de liquide est utilisé par le circuit d'alarme 18 comme un signal de validation du signal émis par le détecteur 6.

Si maintenant, le niveau du sang dans le piège à bulles 1 restant sensiblement le même, le liquide médical cesse de s'écouler dans le piège à bulles 1 (parce que la poche est vide, ou parce que la pompe qui met, le cas échéant, le liquide médical en circulation, s'est arrêtée, ou parce qu'un clamp a été placé sur la canalisation 4, par exemple), le détecteur 6 détecte l'absence de liquide sur la paroi 8 une fois la retenue 5 vidée, et il émet un signal qui est interprété par le circuit d'alarme 18 comme significatif de l'interruption de l'infusion du liquide médical. Une alarme est alors commandée par le circuit d'alarme.

Si maintenant le niveau du sang dans le piège à bulles 1 monte jusqu'à atteindre le détecteur 15, celui-ci émet un signal qui est interprété par le circuit d'alarme 18 comme significatif d'une situation anormale et prime sur le signal émis par le détecteur 6, qui n'est plus pris en compte, quel qu'il soit. En effet, à partir du moment où le sang monte dans le piège à bulles 1, la détection de la présence de liquide par le détecteur 6 ne peut plus être interprétée univoquement comme significative de l'écoulement du liquide médical dans le piège à bulles 1 : le détecteur 6 pourrait aussi bien avoir détecté le sang arrivé à son niveau.

Etant donné la position terminale du piège à bulles 1 dans le circuit de liquide médical qui vient d'être décrit, on notera que le détecteur d'écoulement selon l'invention ne détecte pas seulement l'écoulement du liquide médical dans le piège à bulles mais aussi dans le circuit vasculaire du patient qui est relié directement au circuit de circulation extracorporelle de sang comprenant le piège à bulles 1.

Le récipient représenté sur la figure 3 est un piège à bulles 20, de forme parallélépipédique rectangle, faisant partie d'un circuit de perfusion. Il est relié, par une canalisation 21 connectée à sa partie supérieure, à une poche de liquide médical (non représentée) et, par une canalisation 22 connectée à sa partie inférieure, à un patient.

Ce piège à bulles 20 est équipé d'un détecteur d'écoulement de liquide médical comprenant des moyens de retenue 23 pour le liquide médical dans le piège à bulles 20, constitués par deux parois transversales 24, 25 inclinées délimitant avec deux parois opposées du piège à bulles 20 un entonnoir situé à l'aplomb de la canalisation d'arrivée 21. Les bords supérieurs des parois inclinées 24, 25 ne sont pas jointifs aux parois en vis-à-vis du piège à bulles 20 de façon à ménager deux ouvertures de trop-plein correspondantes 26, 27. Un détecteur 28 de présence du liquide médical dans la retenue 23 est disposé en regard de la base de l'entonnoir juste au-dessus de son orifice d'évacuation 29. Ce détecteur 28 est, par exemple, un détecteur optique du type du détecteur 6 ou du détecteur 15 décrits en relation à la figure 1. Un second détecteur 30, pour détecter la présence de liquide médical dans le piège à bulles 20 est disposé au-dessous du niveau du détecteur 28, à un niveau que le liquide médical n'atteint pas lorsque ce liquide circule en régime de perfusion normal dans le circuit de liquide médical. De même que le détecteur 28, le détecteur 30 est, par exemple, un détecteur optique du type du détecteur 6 ou du détecteur 15 décrits en relation à la figure 1.

Les signaux émis par les deux détecteurs 28, 30 sont reçus et traités dans un circuit d'alarme 18 qui commande une alarme lorsque le détecteur 28 de présence de liquide dans la retenue 23 ne détecte pas de liquide ou lorsque le détecteur 30 de présence de liquide dans le piège à bulles 20 détecte la présence de liquide.

En régime de perfusion normal, du liquide médical, provenant par exemple d'une poche et mis en circulation, le cas échéant, par une pompe, s'écoule par la canalisation 21 dans les moyens de retenue 23 qu'il remplit plus ou moins selon le débit d'entrée du liquide. De là, il s'écoule par l'orifice d'évacuation 29 dans le fond du piège à bulles 20 et, par la canalisation 22, vers le patient. Le détecteur 28 détecte la présence de liquide dans la retenue 23 et le détecteur 30 ne détecte pas la présence de liquide dans le piège à bulles 20, à son niveau, ce qui est interprété par le circuit d'alarme 18 comme significatif de l'écoulement effectif de liquide dans le circuit de liquide médical : le circuit d'alarme ne commande pas d'alarme.

Si pour une raison quelconque, le liquide médical ne s'écoule plus dans la retenue 23 (poche de solution vidée, clamp placé sur la canalisation d'arrivée 21, pompe de circulation disposée en aval du piège à bulles 20, stoppée), une fois cette retenue vidée, aucun des deux détecteurs 28, 30 ne détecte plus la présence de liquide, ce qui est interprété par le circuit d'alarme 18 comme significatif de l'arrêt de l'écoulement du liquide médical, c'est-à-dire aussi l'interruption de la perfusion, et une alarme est émise.

Enfin, si, pour une raison quelconque, la canalisation de sortie 22 est obturée (par un clamp, ou par l'arrêt d'une pompe de circulation disposée en aval du piège à bulles 20), le niveau du liquide monte dans le piège à bulles 20 jusqu'à atteindre le détecteur 30, ce qui est interprété par le circuit d'alarme 18 comme l'interruption de la perfusion : une alarme est émise, que le détecteur 28 détecte ou ne détecte pas la présence de liquide dans la retenue 23.

La présente invention n'est pas limitée aux modes de réalisation qui viennent d'être décrits et elle est susceptible de variantes. Ainsi, bien que dans l'exemple d'utilisation spécifique du détecteur d'écoulement selon l'invention qui a été décrit, les détecteurs de présence de liquide soient disposés à l'extérieur du récipient, les pièges à bulles à usage médical étant généralement à usage unique et ne devant être pollués intérieurement en aucun cas, dans d'autres types d'utilisation, ces détecteurs pourraient être montés sur le récipient de façon à venir au contact du liquide qui s'y écoule.

De même, les détecteurs optiques décrits, qui présentent en général l'avantage d'être fiables et bon marché, pourraient être remplacés par tous détecteurs susceptibles de remplir les mêmes fonctions, détecteurs ultrasoniques par exemple ou, pour le cas où ces détecteurs seraient prévus pour venir au contact du liquide, sondes de conductivité.

Il va de soi en outre que, sur le piège à bulles représenté sur les figures 1 et 2, le détecteur 6 de présence de liquide dans les moyens de retenue 5 pourrait être disposés au-dessus du niveau de l'orifice d'évacuation 9 de la retenue 5, comme c'est le cas sur le piège à bulles représenté sur la figure 3. Et, sur ce dernier, le détecteur 28 de présence de liquide dans les moyens de retenue 23 pourrait être disposés sous l'orifice d'évacuation 29 de la retenue 23. Dans ce cas, l'orifice d'évacuation 29 peut être avantageusement calibré pour provoquer un écoulement goutte à goutte et le détecteur 28, du type comprenant un émetteur et un récepteur de lumière disposés en vis-à-vis interceptant le trajet des gouttes, émet un signal continuellement interrompu tant que la retenue 23 contient du liquide. Pour que la formation de gouttes ne soit pas perturbée lorsque le liquide déborde de la retenue 23, les parois inclinées 24, 25 sont alors munies à leur extrémité supérieure ou inférieure d'un déflecteur, comme le déflecteur 12 représenté sur la figure 1.

## Revendications

1. Dispositif de détection de l'écoulement d'un liquide dans un récipient (1, 20) à usage médical, comprenant des moyens de détection (6, 28) de la présence de liquide dans des moyens de retenue de liquide (5, 23) ayant un orifice d'évacuation (9, 29) à leur base, caractérisé en ce que les moyens de retenue (5, 23) sont situés à l'intérieur du récipient (1, 20) entre un orifice d'entrée (4) de liquide dans le récipient (1, 20) et le fond du récipient et en ce que les moyens de détection (6, 28) sont disposés à l'extérieur du récipient (1, 20) pour détecter tout écoulement de liquide hors des moyens de retenue.

2. Dispositif selon la revendication 1, caractérisé en ce qu'il comprend en outre des moyens de détection (15, 30) de la présence de liquide dans le récipient (1, 20), à un niveau situé au-dessous du niveau des moyens de détection (6, 28) de la présence de liquide dans les moyens de retenue (5, 23).

3. Dispositif selon la revendication 2, caractérisé en ce qu'il comprend des moyens d'alarme (18) pour recevoir des signaux émis par les moyens de détection (6, 28) de la présence de liquide dans les moyens de retenue (5, 23) et des signaux émis par les moyens de détection (15, 30) de la présence de liquide dans le récipient (1, 20), et pour commander une alarme quand ni l'un ni l'autre des détecteurs ne détecte la présence de liquide ou quand les moyens de détection (15, 30) de la présence de liquide dans le récipient (1, 20) détecte la présence de liquide.

4. Dispositif selon une des revendications 1 à 3, caractérisé en ce que les moyens de détection (28) de la présence de liquide dans les moyens de retenue (23) sont disposés en regard du volume délimité par les moyens de retenue (23).

5. Dispositif selon une des revendications 1 à 3, caractérisé en ce que les moyens de détection (6) de la présence de liquide dans les moyens de retenue (5) sont disposés au-dessous du niveau de l'orifice d'évacuation (9) des moyens de retenue (5).

6. Dispositif selon une des revendications 1 à 5, caractérisé en ce que l'orifice d'évacuation (9) des moyens de retenue (5) est disposé contre une paroi latérale (8) du récipient (1).

7. Dispositif selon une des revendications 1 à 5, caractérisé en ce que l'orifice d'évacuation (29) des moyens de retenue (23) est calibré pour provoquer un écoulement goutte à goutte du liquide.

8. Dispositif selon une des revendications 1 à 7, caractérisé en ce qu'il comprend une ouverture de trop-plein (11 ; 26, 27) pour les moyens de retenue de liquide (5, 23).

9. Dispositif selon une des revendications 1 à 8, caractérisé en ce que les moyens de détection (6, 28 ; 15, 30) de la présence de liquide dans les moyens de retenue (5, 23) et/ou le récipient (1, 20) sont des moyens de détection optique.

10. Dispositif selon la revendication 9, caractérisé en ce que les moyens de détection optique (6) comprennent une source (13) et un récepteur (14) de lumière situés à l'extérieur du récipient (1) en vis-à-vis d'une paroi transparente (8) de ce récipient (1) et disposés l'un par rapport à l'autre de façon qu'un rayon émis par la source de lumière (13) se réfléchisse sur la surface interne de cette paroi (8) en direction du récepteur (14) lorsque cette surface n'est pas baignée par un liquide et, lorsque cette surface est baignée par un liquide, soit réfracté dans ce liquide.

11. Dispositif selon une des revendications 1 à 10, caractérisé en ce que le récipient est un piège à bulles (1, 20) faisant partie d'un circuit de perfusion ou d'un circuit de circulation extracorporelle de sang.

## Patentansprüche

1. Vorrichtung zum Ermitteln der Strömung einer Flüssigkeit in einem Behälter (1, 20) zur medizinischen Verwendung, mit einer Ermittlungseinrichtung (6, 28) für die Anwesenheit von Flüssigkeit in einer Flüssigkeitsrückstaueinrichtung (5, 23), die eine Auslaßöffnung (9, 29) an ihrer Basis hat, dadurch gekennzeichnet, daß die Rückstaueinrichtung (5, 23) im Innern des Behälters (1, 20) zwischen einer Flüssigkeitseinlaßöffnung (4) in dem Behälter (1, 20) und dem Boden des Behälters angeordnet ist, und daß die Ermittlungseinrichtung (6, 28) außerhalb des Behälters (1, 20) angeordnet ist, um irgendeine Flüssigkeitsströmung außerhalb der Rückstaueinrichtung zu ermitteln.

2. Vorrichtung nach Anspruch 1, dadurch gekennzeichnet, daß sie außerdem eine Ermittlungseinrichtung (15, 30) für die Anwesenheit von Flüssigkeit in dem Behälter (1, 20) auf einem Niveau umfaßt, das unterhalb des Niveaus der Ermittlungseinrichtung (6, 28) für die Anwesenheit von Flüssigkeit in der Rückstaueinrichtung (5, 23) gelegen ist.

3. Vorrichtung nach Anspruch 2, dadurch gekennzeichnet, daß sie eine Alarmeinrichtung (18) zum Empfangen von Signalen umfaßt, die durch die Ermittlungseinrichtung (6, 28) für die Anwesenheit von Flüssigkeit in der Rückstaueinrichtung (5, 23) ausgegeben werden, und von Signalen, die von der Ermittlungseinrichtung (15, 30) für die Anwesenheit von Flüssigkeit in dem Behälter (1, 20) ausgegeben werden, und zum Auslösen eines Alarms, wenn weder der eine noch der andere der Detektoren die Anwesenheit von Flüssigkeit ermittelt, oder wenn die Ermittlungseinrichtung (15, 30) für die Anwesenheit von Flüssigkeit in dem Behälter (1, 20) die Anwesenheit von Flüssigkeit ermittelt.

4. Vorrichtung nach einem der Ansprüche 1 bis 3, dadurch gekennzeichnet, daß die Ermittlungseinrichtung (28) für die Anwesenheit von Flüssigkeit in der Rückstaueinrichtung (23) gegenüber dem durch die Rückstaueinrichtung (23) begrenzten Volumen angeordnet ist.

5. Vorrichtung nach einem der Ansprüche 1 bis 3, dadurch gekennzeichnet, daß die Ermittlungseinrichtung (6) für die Anwesenheit von Flüssigkeit in der Rückstaueinrichtung (5) oberhalb des Niveaus der Auslaßöffnung (9) der Rückstaueinrichtung (5) angeordnet ist.

6. Vorrichtung nach einem der Ansprüche 1 bis 5, dadurch gekennzeichnet, daß die Auslaßöffnung (9) der Rückstaueinrichtung (5) gegen eine Seitenwand (8) des Behälters (1) angeordnet ist.

7. Vorrichtung nach einem der Ansprüche 1 bis 5, dadurch gekennzeichnet, daß die Auslaßöffnung (29) der Rückstaueinrichtung (23) kalibriert ist, um eine tropfenweise Strömung der Flüssigkeit hervorzurufen.

8. Vorrichtung nach einem der Ansprüche 1 bis 7, dadurch gekennzeichnet, daß sie eine Überlauföffnung (11 ; 26, 27) für die Flüssigkeitsrückstaueinrichtung (5, 23) umfaßt.

9. Vorrichtung nach einem der Ansprüche 1 bis 8, dadurch gekennzeichnet, daß die Ermittlungseinrichtung (6, 28 ; 15, 30) für die Anwesenheit von Flüssigkeit in der Rückstaueinrichtung (5, 23) und/oder dem Behälter (1, 20) eine optische Ermittlungseinrichtung ist.

10. Vorrichtung nach Anspruch 9, dadurch gekennzeichnet, daß die optische Ermittlungseinrichtung (6) eine Lichtquelle (13) und einen Lichtempfänger (14) umfaßt, die außerhalb des Behälters gegenüber einer transparenten Wand (8) des Behälters (1) und in bezug aufeinander derart angeordnet sind, daß ein von der Lichtquelle (13) ausgegebener Strahl auf der Innenfläche dieser Wand (8) in Richtung des Detektors (14) reflektiert wird, wenn diese Fläche nicht durch eine Flüssigkeit benetzt ist, und, wenn diese Fläche durch eine Flüssigkeit benetzt ist, in dieser Flüssigkeit gebrochen wird.

11. Vorrichtung nach einem der Ansprüche 1 bis 10, dadurch gekennzeichnet, daß der Behälter eine Blasenfalle (1, 20) ist, der einen Teil einer Perfusionsleitung oder einer extrakorporellen Blutkreislaufleitung ist.

## Claims

1. Device for detecting the flow of a liquid in a receptacle (1, 20) for medical use, comprising means (6, 28) for detecting the presence of liquid in a liquid retention means (5, 23) having a drain orifice (9, 29) at its base, characterized in that the retention means (5, 23) is situated within the receptacle (1, 20) between an orifice (4) for entry of liquid into the receptacle (1, 20) and the bottom of the receptacle, and in that the detection means (6, 28) is arranged outside the receptacle (1, 20) to detect any flow of liquid out of the retention means.

2. Device according to Claim 1, characterized in that it further comprises means (15, 30) for detecting the presence of liquid in the receptacle (1, 20), at a level situated below the level of the means (6, 28) for detecting the presence of liquid in the retention means (5, 23).

3. Device according to Claim 2, characterized in that it comprises alarm means (18) for receiving signals sent by the means (6, 28) for detecting the presence of liquid in the retention means (5, 23) and signals sent by the means (15, 30) for detecting the presence of liquid in the receptacle (1, 20), and for raising an alarm when neither of the two detectors detects the presence of liquid or when the means (15, 30) for detecting the presence of liquid in the receptacle (1, 20) detects the presence of liquid.

4. Device according to one of Claims 1 to 3, characterized in that the means (28) for detecting the presence of liquid in the retention means (23) is located opposite the volume delimited by the retention means (23).

5. Device according to one of Claims 1 to 3, characterized in that the means (6) for detecting the presence of liquid in the retention means (5) is located below the level of the drain orifice (9) of the retention means (5).

6. Device according to one of Claims 1 to 5, characterized in that the drain orifice (9) of the retention means (5) is located against a side wall (8) of the receptacle (1).

7. Device according to one of Claims 1 to 5, characterized in that the drain orifice (29) of the retention means (23) is calibrated to cause a drop-by-drop flow of the liquid.

8. Device according to one of Claims 1 to 7, characterized in that it comprises an overflow opening (11; 26, 27) for the liquid retention means (5, 23).

9. Device according to one of Claims 1 to 8, characterized in that the means (6, 28; 15, 30) for detecting the presence of liquid in the retention means (5, 23) and/or the receptacle (1, 20) is optical detection means.

10. Device according to Claim 9, characterized in that the optical detection means (6) comprises a light source (13) and a light receiver (14) situated outside the receptacle (1) face to face with a transparent wall (8) of this receptacle (1) and arranged with respect to one another such that a ray emitted by the light source (13) is reflected on the inner surface of this wall (8) towards the receiver (14) when this surface is not bathed in a liquid and, when this surface is bathed in a liquid, is refracted in this liquid.

11. Device according to one of Claims 1 to 10, characterized in that the receptacle is a bubble trap (1, 20) forming part of an infusion circuit or of an extracorporeal blood circulation circuit.
